# EUROPEAN PATENT APPLICATION

(11) **EP 4 279 596 A1**
(43) Date of publication of application: **22.11.2023**
(21) Application number: 21919587.2
(22) Date of filing: 30.11.2021
(51) Int. Cl.: C12N 15/62, A61K 35/12, A61K 35/15, A61K 39/395, A61P 31/00, A61P 35/00, A61P 37/06, A61P 37/08, C07K 14/705, C07K 16/18, C07K 16/28, C07K 16/30, C07K 16/32, C07K 19/00, C12N 5/0784, C12N 5/0786, C12N 5/10, C12N 15/12, C12N 15/13

(54) **CHIMERIC TARGET FACTOR RECEPTOR**

(30) Priority: 15.01.2021 JP 2021005337
(71) Applicant: National University Corporation Tokai National Higher Education and Research System, Nagoya-shi, Aichi 464-8601 (JP)
(72) Inventor: KASUYA, Hideki, Nagoya-shi, Aichi 464-8601 (JP); NAOE, Yoshinori, Nagoya-shi, Aichi 464-8601 (JP); MATSUMURA, Shigeru, Nagoya-shi, Aichi 464-8601 (JP); EISSA, Ibrahim Ragab Nassr, Nagoya-shi, Aichi 464-8601 (JP); ALI MOHAMED, Mohamed Abdelmoneim Ahmed, Nagoya-shi, Aichi 464-8601 (JP)
(74) Representative: Hoffmann Eitle
(86) International application number: PCT/JP2021/043978
(87) International publication number: WO 2022/153698

(57) **Abstract**

Provided is a chimeric target factor receptor capable of activating an antigen-presenting cell such as a dendritic cell in a target-factor-specific manner. The chimeric target factor receptor contains a target factor-binding domain, a transmembrane domain, and an intracellular domain containing a TLR intracellular domain.

## Description

### Technical Field

The present invention relates to chimeric target factor receptors.

### Background Art

A chimeric antigen receptor binds to an antigen according to antibody specificity and thereby causes target-cell damage, cytokine release, and T-cell division after stimulation. Gene transfer can impart specificity to T cells, and the preparation of such cells is generally easier than culture amplification of T cells positive for endogenous tumor-specific T-cell receptors. Additionally, therapies using a chimeric antigen receptor (CAR-T therapies) can exhibit higher cytotoxic activity than antibody therapies.

Dendritic cells are immune cells that play a role in innate immune activation by recognizing microorganism-specific antigens, such as bacteria and viruses. With a focus on this immune activation ability, attempts have been made to return dendritic cells that have once recognized cancer antigens to the body (dendritic cell vaccine therapies).

### Citation List

### Patent Literature

PTL 1: US 2017/0151281 A1

### Summary of Invention

### Technical Problem

PTL 1 reports a CAR-DC composed of a dendritic cell and a chimeric antigen receptor incorporated into the cell. However, there is no report on the details of the structure of the intracellular domain.

An object of the present invention is to provide a chimeric target factor receptor capable of activating antigen-presenting cells, such as dendritic cells, in a target-factor-specific manner.

### Solution to Problem

The present inventors conducted extensive research in view of the above problem and found that the object can be achieved by a chimeric target factor receptor containing a target factor-binding domain, a transmembrane domain, and an intracellular domain including a TLR intracellular domain. As a result of further research based on this finding, the inventors completed the present invention. Specifically, the present invention includes the following aspects.

Item 1. A chimeric target factor receptor comprising
a target factor-binding domain,
a transmembrane domain, and
an intracellular domain containing a TLR intracellular domain.

Item 2. The chimeric target factor receptor according to Item 1, wherein the TLR intracellular domain is at least one selected from the group consisting of a TLR2 intracellular domain, a TLR3 intracellular domain, and a TLR9 intracellular domain.

Item 3. The chimeric target factor receptor according to Item 1 or 2, wherein the TLR intracellular domain is at least one selected from the group consisting of a TLR3 intracellular domain and a TLR9 intracellular domain.

Item 4. The chimeric target factor receptor according to any one of Items 1 to 3, wherein the intracellular domain further contains a CD40 intracellular domain.

Item 5. The chimeric target factor receptor according to any one of Items 1 to 4, wherein the target factor-binding domain has a single-chain antibody structure.

Item 6. The chimeric target factor receptor according to any one of Items 1 to 5, wherein the target factor is at least one selected from the group consisting of cancer antigens, cytokine receptors, and cell growth factor receptors.

Item 7. A polynucleotide encoding the chimeric target factor receptor of any one of Items 1 to 6.

Item 8. A cell comprising the polynucleotide of Item 7.

Item 9. The cell according to Item 8, which is an antigen-presenting cell.

Item 10. The cell according to Item 8 or 9, which is a dendritic cell.

Item 11. A pharmaceutical composition comprising the cell of any one of Items 8 to 10.

Item 12. The pharmaceutical composition according to Item 11, which is for use in the treatment, prevention, or amelioration of at least one selected from the group consisting of cancers, autoimmune diseases, allergic diseases, and infectious diseases.

Item 12A. A method for treating, preventing, or ameliorating at least one selected from the group consisting of cancers, autoimmune diseases, allergic diseases, and infectious diseases,
the method comprising administering the cell of any one of Items 8 to 10 to a subject or patient with at least one selected from the group consisting of cancers, autoimmune diseases, allergic diseases, and infectious diseases.

Item 12B. The cell according to any one of Items 8 to 10, which is for use in a pharmaceutical composition for the treatment, prevention, or amelioration of at least one selected from the group consisting of cancers, autoimmune diseases, allergic diseases, and infectious diseases.

Item 12C. Use of the cell of any one of Items 8 to 10 in the production of a pharmaceutical composition for the treatment, prevention, or amelioration of at least one selected from the group consisting of cancers, autoimmune diseases, allergic diseases, and infectious diseases.

Item 13. The pharmaceutical composition according to Item 11 or 12, which is for use in the treatment, prevention, or amelioration of solid cancer.

Item 14. The pharmaceutical composition according to Item 13, wherein
the TLR intracellular domain is at least one selected from the group consisting of a TLR3 intracellular domain and a TLR9 intracellular domain, and
the cell is a dendritic cell.

Item 15. The pharmaceutical composition according to Item 13 or 14,
wherein
the intracellular domain further contains a CD40 intracellular domain, and
the cell is a dendritic cell.

Item 16. The pharmaceutical composition according to any one of Items 11 to 15, which is for use in combination with an immune checkpoint inhibitor.

### Advantageous Effects of Invention

The present invention provides a chimeric target factor receptor capable of activating an antigen-presenting cell, such as a dendritic cell, in a target-factor-specific manner. Additionally, the present invention provides a polynucleotide encoding the chimeric target factor receptor, a cell containing the polynucleotide, a pharmaceutical composition containing the cell, and the like.

### Brief Description of Drawings

Fig. 1 shows the results of an in vitro dendritic cell activation assay (Test Example 4, co-culture for 5 hours, index factor: CD80). The caption "With TLR" indicates the result of using the TLR intracellular domain shown in the upper left of the graphs for the intracellular domain, and the caption "Without TLR" indicates the result of using no intracellular domain. The horizontal axis represents the expression level of CD80, and the vertical axis represents the number of cells.
Fig. 2 shows the results of an in vitro dendritic cell activation assay (Test Example 4, co-culture for 24 hours, index factor: CD80). The captions are as explained in Fig. 1.
Fig. 3 shows the results of an in vitro dendritic cell activation assay (Test Example 4, co-culture for 48 hours, index factor: CD80). The captions are as explained in Fig. 1.
Fig. 4 shows the results of an in vitro dendritic cell activation assay (Test Example 4, co-culture for 5 hours, index factor: CD86). The captions are as explained in Fig. 1.

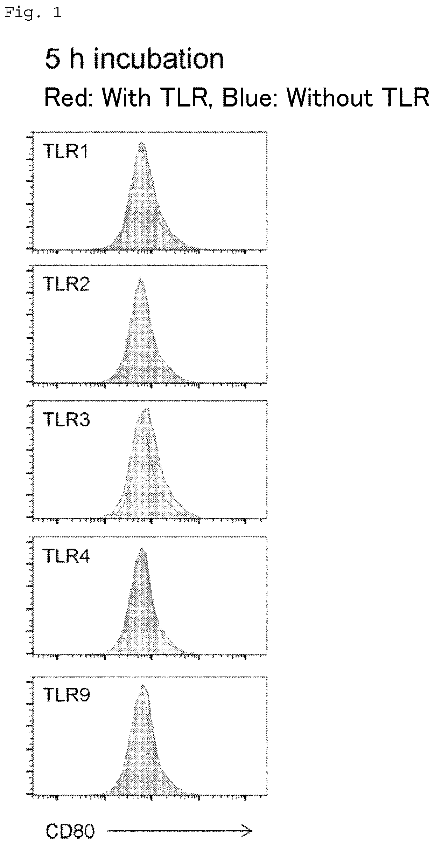

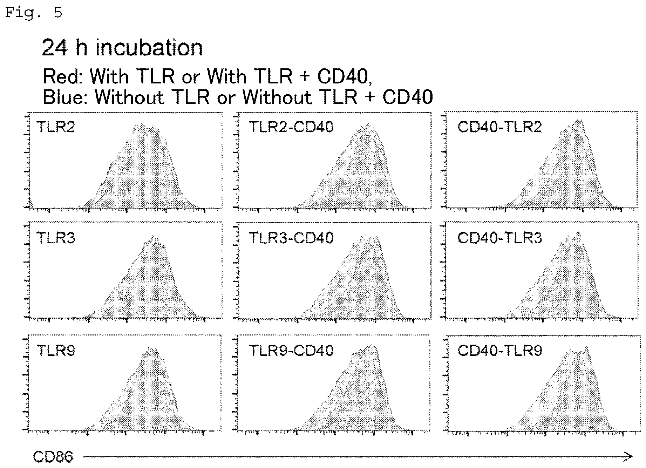

Fig. 5 shows the results of an in vitro dendritic cell activation assay (Test Example 4, co-culture for 24 hours, index factor: CD86). In the graphs in the leftmost column, the caption "With TLR" indicates the result of using the TLR intracellular domain shown in the upper left of the graphs for the intracellular domain, and the caption "Without TLR" indicates the result of using no intracellular domain. In the graphs in the middle column and the rightmost column, the caption "With TLR + CD40" indicates the result of using the TLR intracellular domain shown in the upper left of the graphs and the intracellular domain of CD40 for the intracellular domain (In the view, the left side indicates the cell membrane side). The caption "Without TLR + CD40" indicates the result of using no intracellular domain. The vertical axis and the horizontal axis are as explained in Fig. 1.
Fig. 6 shows the results of an in vitro macrophage phagocytosis assay (Test Example 5). The horizontal axis represents the intracellular domain, and the symbol "Δ" represents the case of using no intracellular domain. The vertical axis represents the phagocytic capacity of macrophages.

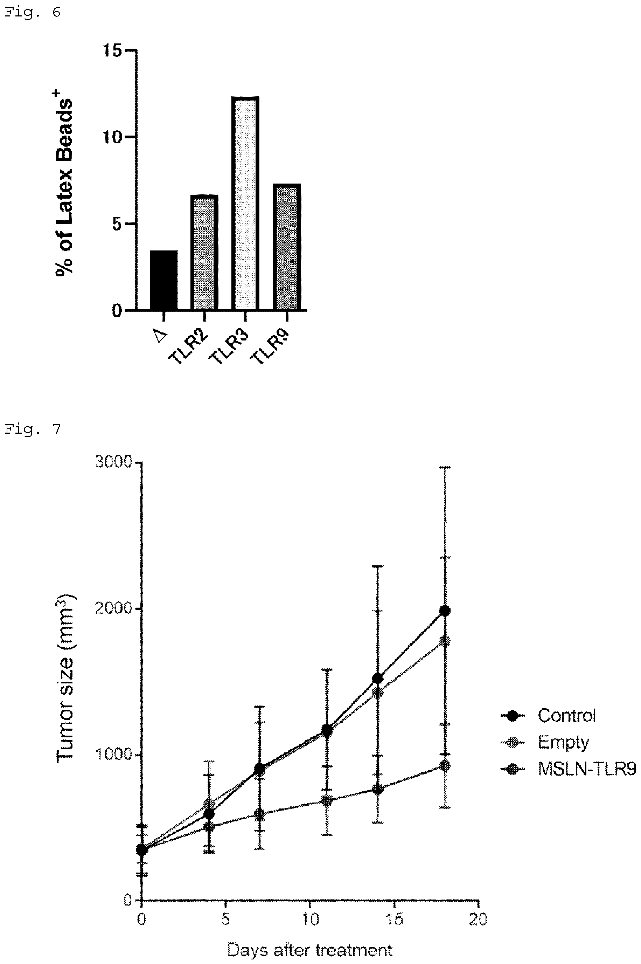

Fig. 7 shows the results of an antitumor effect evaluation test (Test Example 6: using mice transplanted with MSLN-expressing cells (Pan02/MSLN)). "Empty" indicates the case of administration of dendritic cells having an empty vector (pMXs-GFP) introduced. "MSLN-TLR9" indicates the case of administration of chimeric target factor receptor-expressing dendritic cells (Test Example 2: the target is MSLN, and the intracellular domain is of TLR9). "Control" indicates the case of administration of no cells. The vertical axis represents the tumor size, and the horizontal axis represents the number of days elapsed from the first administration.
Fig. 8 shows the results of an antitumor effect evaluation test (Test Example 6: using mice transplanted with MSLN-non-expressing cells (Pan02)). The captions are as explained in Fig. 7.
Fig. 9 shows the results of an in vitro dendritic cell activation assay (Test Example 7, index factor: MHC class I). The captions are as explained in Fig. 1.
Fig. 10 shows the results of an in vitro dendritic cell activation assay (Test Example 7, index factor: MHC class II). The captions are as explained in Fig. 1.
Fig. 11 shows the results of an in vitro dendritic cell activation assay (Test Example 8, index factor: CD86). The captions are as explained in Fig. 1.
Fig. 12 shows the results of an in vitro dendritic cell activation assay (Test Example 8, index factor: MHC class II). The captions are as explained in Fig. 5.
Fig. 13 shows the results of an antitumor effect evaluation test (Test Example 9, using mice transplanted with MSLN-expressing cells (Pan02/MSLN)). "Control" indicates the case of administration of no cells, and the other captions indicate the target (MSLN) and the intracellular domain of the chimeric target factor receptor (e.g., "TLR9" indicates the intracellular domain of TLR9, and "Δ" indicates the absence of the intracellular domain). The vertical axis represents the tumor size, and the horizontal axis represents the number of days elapsed from the first administration.
Fig. 14 shows the results of an antitumor effect evaluation test (Test Example 11, using mice transplanted with MSLN-expressing cells (Pan02/MSLN)). "Control" indicates the case of administration of no cells, and the other captions indicate the target (MSLN) and the intracellular domain of the chimeric target factor receptor (e.g., "TLR9" indicates the intracellular domain of TLR9). The vertical axis represents the tumor size, and the horizontal axis represents the number of days elapsed from the first administration.

### Description of Embodiments

### 1. Definition

In the present specification, the terms "comprise," "contain," and "include" include the concepts of comprising, containing, consisting essentially of, and consisting of.

The "identity" of amino acid sequences refers to the degree to which two or more contrastable amino acid sequences match each other. Thus, the higher the degree of match between two amino acid sequences, the higher the identity or similarity of those sequences. The level of amino acid sequence identity is determined, for example, by using FASTA, which is a tool for sequence analysis, with default parameters. Alternatively, the level of amino acid sequence identity can be determined by using the BLAST algorithm by Karlin and Altschul (Karlin S, Altschul SF. Methods for assessing the statistical significance of molecular sequence features by using general scoring schemes, Proc Natl Acad Sci USA. 87: 2264-2268 (1990); and Karlin S, Altschul SF. Applications and statistics for multiple high-scoring segments in molecular sequences, Proc Natl Acad Sci USA. 90: 5873-7 (1993)). A program called "BLASTX," based on this BLAST algorithm, has been developed. Specific procedures of these analysis methods are known and can be found on the website of the National Center of Biotechnology Information (NCBI) (http://www.ncbi.nlm.nih.gov/). The identity of base sequences is also defined in the same manner as above.

In the present specification, "conservative substitution" means the substitution of an amino acid residue with another amino acid residue having a similar side chain. For example, the substitution between amino acid residues having a basic side chain, such as lysine, arginine, or histidine, is considered to be a conservative substitution. The following substitutions between other amino acid residues are also considered to be a conservative substitution: the substitution between amino acid residues having an acidic side chain such as aspartic acid and glutamic acid; the substitution between amino acid residues having an uncharged polar side chain such as glycine, asparagine, glutamine, serine, threonine, tyrosine, or cysteine; the substitution between amino acid residues having a nonpolar side chain such as alanine, valine, leucine, isoleucine, proline, phenylalanine, methionine, or tryptophan; the substitution between amino acid residues having a β-branched side chain such as threonine, valine, or isoleucine; and the substitution between amino acid residues having an aromatic side chain such as tyrosine, phenylalanine, tryptophan, or histidine.

In the present specification, "CDR" is an abbreviation for complementarity determining region. A CDR is a region present in the variable regions of immunoglobulins or T-cell receptors and is deeply involved in the specific binding of an antibody or T-cell receptor to an antigen. The phrase "light-chain CDR" refers to a CDR present in the light-chain variable regions of immunoglobulins, and the phrase "heavy-chain CDR" refers to a CDR present in the heavy-chain variable regions of immunoglobulins. In T-cell receptors, there are also α chain, β chain, γ chain, δ chain, and like chains, and the same applies to the CDRs of these.

In the present specification, the phrase "variable region" refers to a region containing CDR1 to CDR3 (simply "CDRs 1-3" below). The order in which CDRs 1-3 are arranged is not particularly limited; however, the variable region preferably refers to a region in which CDR1, CDR2, and CDR3 are arranged in this order in the direction from the N-terminus toward the C-terminus or in the reverse order either consecutively or via other amino acid sequences referred to as "framework regions" (FRs), described later. The phrase "heavy-chain variable region" refers to a region of immunoglobulins in which heavy-chain CDRs 1-3 are arranged, and the phrase "light-chain variable region" refers to a region of immunoglobulins in which light-chain CDRs 1-3 are arranged. In T-cell receptors, there are also α chain, β chain, γ chain, δ chain, and like chains, and the same applies to the CDRs of these.

The regions other than CDRs 1-3 of each variable region are referred to as "framework regions" (FRs), as mentioned above. In particular, the region between the N-terminus and CDR1 of a variable region is defined as FR1, the region between CDR1 and CDR2 as FR2, the region between CDR2 and CDR3 as FR3, and the region between CDR3 and the C-terminus of a variable region as FR4 .

### 2. Chimeric Target Factor Receptor

In an embodiment, the present invention relates to a chimeric target factor receptor containing a target factor-binding domain, a transmembrane domain, and an intracellular domain including a TLR intracellular domain (which may be referred to as "the chimeric target factor receptor of the present invention" in the present specification). The following explains the chimeric target factor receptor.

The target factor-binding domain is not particularly limited as long as the target factor-binding domain is extracellularly arranged when the chimeric target factor receptor of the present invention is placed on the cell membrane, and as long as the target factor-binding domain can recognize and bind (preferably specifically) to a target factor. Examples of target factors include cancer antigens, various receptors (e.g., cytokine receptors such as interleukin receptors, and cell growth factor receptors), cell adhesion factors, and membrane proteins of bacteria or viruses. Specific examples of target factors include CD19, GD2, GD3, CD20, CD37, CEA, HER2, EGFR, type III mutant EGFR, CD38, BCMA, MUC-1, PSMA, WT1, cancer testis antigens (e.g., NY-ESO-1, and MAGE-A4), mutation peptides (e.g., k-ras, h-ras, and p53), hTERT, PRAM, TYRP1, mesothelin, PMEL, mucin, IL-12R, IL-4R, IL-13R, IL-6R, IL-23R, CTLA4, EGFR806, PSCA, Claudin 18.2, EpCAM, VEGFR2, Nectin4/FAP, LewisY, Glypican-3, IL-13Rα2, CD171, MUC16, AFP, AXL, CD80/86, c-MET, DLL-3, DR5, EpHA2, FR-α, gp100, MAGE-A1/3/4, and LMP1. Examples of target factor-binding domains include a domain containing one or more CDRs of an antibody or T-cell receptor, a domain containing all or part of a cytokine or cell growth factor (a cytokine or cell growth factor domain), and a domain containing all or part of a cell adhesion factor (a cell adhesion factor domain). A domain containing one or more CDRs of an antibody or T-cell receptor typically has one or more, preferably two, three, four, five or more, more preferably all six of the CDRs of the antibody or T-cell receptor against a target factor (e.g., if the CDRs of an antibody are the case, heavy-chain CDR1, heavy-chain CDR2, heavy-chain CDR3, light-chain CDR1, light-chain CDR2, and light-chain CDR3). The domain containing one or more CDRs more preferably contains a variable region of an antibody or T-cell receptor against a target factor (if an antibody is the case, the domain contains, for example, a heavy-chain variable region and/or a light-chain variable region). Examples of cytokines or cell growth factor domains include a domain containing a domain bindable to a receptor, such as IL-12, IL-4, IL-13, IL-6, IL-23, or GM-CSF.

When containing one or more CDRs, the target factor-binding domain preferably has a single-chain antibody structure, and more preferably the scFv structure. When the target factor-binding domain contains a heavy-chain variable region and a light-chain variable region as in the scFv structure, the heavy-chain variable region and the light-chain variable region are typically connected via a linker. Any linker can be used as long as target-factor bindable properties are not significantly impaired. The linker is preferably glycine or a linker composed of glycine and serine (e.g., GGS linker, GS linker, and GGG linker). The length of the linker is not particularly limited. The number of amino acid residues of the linker is, for example, 5 to 30. Arrangement of the heavy-chain variable region and the light-chain variable region is not particularly limited. The light-chain variable region may be at a position closer to the N-terminus, or the heavy-chain variable region may be at a position closer to the N-terminus.

The transmembrane domain is not particularly limited as long as the transmembrane domain is placed inside a cell membrane when the chimeric target factor receptor of the present invention is placed on the cell membrane, and as long as the transmembrane domain can compose the chimeric target factor receptor. Examples of transmembrane domains for use include transmembrane regions of CD28, CD3ε, CD8α, CD3, CD4, CD4-1BB, etc. Transmembrane regions are known, or can be easily determined based on known sequence information (e.g., using a program to predict a transmembrane region). Alternatively, a transmembrane domain formed of an artificially constructed polypeptide may be used. These transmembrane domains may be mutated as long as the functionality of the chimeric target factor receptor is not significantly impaired.

The transmembrane domain for use may be preferably the transmembrane region of CD28. Specific examples of the transmembrane region of CD28 include the amino acid sequence described in the following (a) and the amino acid sequence described in the following (b):
(a) The amino acid sequence of SEQ ID NO: 2; and
(b) An amino acid sequence that has at least 85% identity to the amino acid sequence of SEQ ID NO: 2 and that can be placed inside a cell membrane when the chimeric target factor receptor of the present invention is placed on the cell membrane.

In item (b) above, the identity is preferably at least 90%, more preferably at least 95%, still more preferably at least 98%, and particularly preferably at least 99%.

Examples of the amino acid sequence described in item (b) include: (b') an amino acid sequence that has the substitution, deletion, addition, or insertion of one or multiple amino acids in the amino acid sequence of SEQ ID NO: 3, and that can be placed inside a cell membrane when the chimeric target factor receptor of the present invention is placed on the cell membrane.

In item (b'), "multiple amino acids" means, for example, 2 to 5 amino acids, preferably 2 or 3 amino acids, and more preferably 2 amino acids.

The target factor-binding domain and the transmembrane domain are linked directly or via a spacer. The sequence of the spacer is not particularly limited; examples of the spacer for use include the sequence of the hinge region or portion thereof of IgG, preferably human IgG (e.g., subtype IgG1 or IgG4), the sequence of the hinge region and a portion of CH2, and the sequence of a portion of a factor used in the transmembrane domain (e.g., CD28). It is likely that the use of the sequence of a hinge region or a portion thereof forms a flexible spacer.

The intracellular domain is placed intracellularly when the chimeric target factor receptor of the present invention is placed on a cell membrane, and the intracellular domain is capable of transmitting a signal necessary for activation of an antigen-presenting cell. In the chimeric target factor receptor of the present invention, the intracellular domain contains a TLR intracellular domain.

The TLR intracellular domain can be any domain containing the amino acid sequence of the intracellular domain of a TLR (Toll-like receptor).

TLRs for use may be those derived from various mammals, such as humans, monkeys, mice, rats, dogs, cats, and rabbits. Of these, TLRs derived from a target organism (e.g., humans) are preferable. The amino acid sequences of TLR intracellular domains derived from various organisms are known.

Examples of TLRs include TLR1 (the amino acid sequence of the intracellular domain is, for example, SEQ ID NO: 3), TLR2 (the amino acid sequence of the intracellular domain is, for example, SEQ ID NO: 4), TLR3 (the amino acid sequence of the intracellular domain is, for example, SEQ ID NO: 5), TLR4 (the amino acid sequence of the intracellular domain is, for example, SEQ ID NO: 6), and TLR9 (the amino acid sequence of the intracellular domain is, for example, SEQ ID NO: 7). Of these, for example, TLR2, TLR3, and TLR9 are preferable, TLR3 and TLR9 are more preferable, and TLR3 is still more preferable, from the viewpoint of their ability to activate antigen-presenting cells, and their ability to activate antigen-presenting cells earlier and more persistently in response to a target factor. In a preferred embodiment of the present invention, the TLR intracellular domain is at least one selected from the group consisting of a TLR2 intracellular domain, a TLR3 intracellular domain, and a TLR9 intracellular domain.

The TLR intracellular domain may have mutation such as substitution, deletion, addition, or insertion of amino acids as long as the TLR intracellular domain has action of activating antigen-presenting cells. The mutation is preferably substitution, more preferably conservative substitution, from the viewpoint that the above action is less likely to be impaired.

A preferred specific example of the amino acid sequence of the TLR intracellular domain is at least one selected from the group consisting of the amino acid sequence described in the following (c) and the amino acid sequence described in the following (d):
(c) the amino acid sequence of any one of SEQ ID NOs: 3 to 7; and
(d) an amino acid sequence that has at least 85% identity to the amino acid sequence of any one of SEQ ID NOs: 3 to 7 and that can constitute a protein having action of activating antigen-presenting cells.

In item (d), the identity is preferably at least 90%, more preferably at least 95%, still more preferably at least 98%, and particularly preferably at least 99%.

Examples of the amino acid sequence described in item (d) include (d') an amino acid sequence that has the substitution, deletion, addition, or insertion of one or multiple amino acids in the amino acid sequence of SEQ ID NO: 4, and that has action of activating antigen-presenting cells.

In item (d'), "multiple amino acids" means, for example, 2 to 5 amino acids, preferably 2 or 3 amino acids, and more preferably 2 amino acids.

The intracellular domain may preferably further contain a CD40 intracellular domain from the viewpoint of the ability to activate antigen-presenting cells, and the ability to activate antigen-presenting cells earlier and more persistently in response to a target factor.

The CD40 intracellular domain can be any domain containing the amino acid sequence of the intracellular domain of the amino acids of CD40.

CD40 for use may be those derived from various mammals, such as humans, monkeys, mice, rats, dogs, cats, and rabbits. Of these, CD40 derived from a target organism (e.g. humans) is preferable.

The amino acid sequences of CD40 intracellular domains from various organisms are known. Specific examples include the amino acid sequence of SEQ ID NO: 8.

The CD40 intracellular domain may have mutation such as the substitution, deletion, addition, or insertion of amino acids as long as the CD40 intracellular domain has action of activating antigen-presenting cells. The mutation is preferably substitution, and more preferably conservative substitution, from the viewpoint that the above action is less likely to be impaired.

A preferred specific example of the amino acid sequence of the CD40 intracellular domain is at least one selected from the group consisting of the amino acid sequence described in the following (e) and the amino acid sequence described in the following (f):
(e) the amino acid sequence of SEQ ID NO: 8; and
(f) an amino acid sequence that has at least 85% identity to the amino acid sequence of SEQ ID NO: 8 and that can constitute a protein having action of activating antigen-presenting cells.

In item (f), the identity is preferably at least 90%, more preferably at least 95%, still more preferably at least 98%, and particularly preferably at least 99%.

Examples of the amino acid sequence described in item (f) include
(f') an amino acid sequence that has the substitution, deletion, addition, or insertion of one or multiple amino acids in the amino acid sequence of SEQ ID NO: 8, and that has action of activating antigen-presenting cells.

In item (f'), "multiple amino acids" means, for example, 2 to 5 amino acids, preferably 2 or 3 amino acids, and more preferably 2 amino acids.

In the intracellular domain containing a CD40 intracellular domain, arrangement of the TLR intracellular domain and the CD40 intracellular domain is not particularly limited. The TLR intracellular domain may be at a position closer to the N-terminus, or the CD40 intracellular domain may be at a position closer to the N-terminus. The TLR intracellular domain and the CD40 intracellular domain are preferably connected directly or via a linker. The linker is not particularly limited and can be any linker. The linker is preferably glycine or a linker composed of glycine and serine (e.g., GGS linker, GS linker, and GGG linker). The length of the linker is not particularly limited. The number of amino acid residues of the linker is, for example, 1 to 20, preferably 1 to 10, and more preferably 1 to 5.

The chimeric target factor receptor of the present invention may contain regions other than those described above. Examples of other regions include intracellular domains such as CD3ζ and FcεRIγ, a leader sequence (signal peptide) for promoting the transport of a CAR to a cell membrane (e.g., the leader sequence of the GM-CSF receptor), and a spacer or linker (e.g., between the transmembrane region and the intracellular signal domain or between domains within the intracellular domain).

In the chimeric target factor receptor of the present invention, the target factor-binding domain, the transmembrane domain, and the intracellular domain are arranged in this order.

The chimeric target factor receptor of the present invention may be a molecule formed of a single type of polypeptide or a molecule formed of a complex of two or more types of polypeptides. Additionally, the chimeric target factor receptor of the present invention may also be a molecule formed of a polypeptide or of a complex of polypeptides, or a molecule formed of a polypeptide or complex of polypeptides to which another substance (e.g., a fluorescent substance, a radioactive substance, or an inorganic particle) is linked.

The chimeric target factor receptor of the present invention may be chemically modified. The polypeptide constituting the chimeric target factor receptor of the present invention may have a carboxyl group (-COOH), carboxylate (-COO-), amide (-CONH₂), or ester (-COOR) at the C-terminus. "R" in the ester is, for example, a C₁₋₆ alkyl group, such as methyl, ethyl, n-propyl, isopropyl, or n-butyl; a C₃₋₈ cycloalkyl group, such as cyclopentyl or cyclohexyl; a C₆₋₁₂ aryl group, such as phenyl or α-naphthyl; a phenyl-C₁₋₂ alkyl group, such as benzyl or phenethyl; a C₇₋₁₄ aralkyl group, such as an α-naphthyl-C₁₋₂ alkyl group, such as α-naphthyl methyl; or a pivaloyloxymethyl group. The polypeptide constituting the chimeric target factor receptor of the present invention may have an amidated or esterified carboxyl group (or carboxylate), which is not the carboxyl group at the C-terminus. The ester in this case may be, for example, the esters of the C-terminus described above. The polypeptide constituting the chimeric target factor receptor of the present invention further includes polypeptides having the amino group of the N-terminal amino acid residue protected by a protective group (e.g., a C₁₋₆ acyl group, including a C₁₋₆ alkanoyl, such as a formyl group and an acetyl group), polypeptides having the N-terminal glutamine residue (which are formed due to cleavage in vivo) pyroglutamated; and polypeptides having a substituent (e.g., -OH, -SH, an amino group, an imidazole group, an indole group, and a guanidino group) on the side chain of an amino acid in the molecule protected by an appropriate protective group (e.g., a
C₁₋₆ acyl group, including a C₁₋₆ alkanoyl group, such as a formyl group and an acetyl group).

The chimeric target factor receptor of the present invention may have a protein or peptide (e.g., a known protein tag or signal sequence) added. Examples of protein tags include biotin, a His tag, a FLAG tag, a Halo tag, an MBP tag, an HA tag, a Myc tag, a V5 tag, a PA tag, and a fluorescent protein tag.

The chimeric target factor receptor of the present invention may be in the form of a pharmaceutically acceptable salt formed with an acid or base. The salt can be any pharmaceutically acceptable salt, and can be either an acid salt or a basic salt. Examples of acid salts include inorganic acid salts, such as hydrochloride, hydrobromide, sulfate, nitrate, and phosphate; organic acid salts, such as acetate, propionate, tartarate, fumarate, maleate, malate, citrate, methanesulfonate, and para-toluenesulfonate; and amino acid salts, such as aspartate and glutamate. Examples of basic salts include alkali metal salts, such as sodium salts and potassium salts; and alkaline-earth metal salts, such as calcium salts and magnesium salts.

The chimeric target factor receptor of the present invention may be in the form of a solvate. The solvent can be any pharmaceutically acceptable solvent, and may be, for example, water, ethanol, glycerol, or acetic acid.

The techniques for producing a chimeric target factor receptor and a cell that expresses a chimeric target factor receptor are known. These can be produced in accordance with a known method or an equivalent method.

### 3. Polynucleotide and Use Thereof

In an embodiment, the present invention relates to a polynucleotide encoding the chimeric target factor receptor of the present invention (which may be referred to as "the polynucleotide of the present invention" in the present specification). The following describes the polynucleotide.

The polynucleotide of the present invention may contain other sequences in addition to the coding sequence of the chimeric target factor receptor of the present invention. The polynucleotide of the present invention preferably contains the chimeric target factor receptor of the present invention in an expressible state. Other sequences include promoter sequences, enhancer sequences, repressor sequences, insulator sequences, origin of replication, coding sequences for reporter proteins (e.g., fluorescent proteins), and coding sequences for drug-resistant genes.

The polynucleotide of the invention preferably contains an expression cassette of the chimeric target factor receptor of the present invention. The expression cassette contains a promoter and a coding sequence for the chimeric target factor receptor of the present invention under control of the promoter. The coding sequence is usually placed downstream of the promoter so as to be under control of the promoter. Examples of promoters usable in the expression cassette include CMV-IE (cytomegalovirus early gene-derived promoter), SV40ori, retroviral LTP, SRα, EF1α, and β-actin promoters. The promoter is operably linked to the coding sequence. The phrase "the promoter is operably linked to the coding sequence" is synonymous with the phrase "the coding sequence is placed under control of the promoter." Typically, the coding sequence is linked at the 3' end of the promoter directly or via another sequence. A poly-A addition signal sequence is placed downstream of the coding sequence. Transcription is ended by using a poly-A addition signal sequence. The poly-A addition signal sequence for use may be, for example, the poly-A addition sequence of SV40 or the poly-A addition sequence of a bovine growth hormone gene.

The expression cassette may contain, for example, a detection gene (a reporter gene, a cell-specific or tissue-specific gene, a selection marker gene, etc.), an enhancer sequence, and a WRPE sequence. The detection gene is used in determining the success or failure, or efficiency of the introduction of the expression cassette, detecting the expression of the CAR gene, determining the expression efficiency of the CAR gene, or selecting and collecting cells expressing the CAR gene. The use of an enhancer sequence improves expression efficiency. The detection gene for use includes the neo gene that confers resistance to neomycin, the npt gene (Herrera Estrella, EMBO J. 2 (1983), 987-995) and the nptII gene (Messing & Vierra. Gene 1 9: 259-268 (1982)) that confer resistance to kanamycin etc., the hph gene that confers resistance to hygromycin (Blochinger & Diggelmann, Mol Cell Bio 4: 2929-2931), the dhfr gene that confers resistance to methotrexate (Bourouis et al., EMBO J. 2 (7)), etc. (marker genes); genes of fluorescent proteins such as luciferase genes (Giacomin, P1. Sci. 116 (1996), 59 to 72; Scikantha, J. Bact. 178 (1996) 121), β-glucuronidase (GUS) gene, GFP (Gerdes, FEBS Lett. 389 (1996), 44-47), and variants thereof (e.g., EGFP and d2EGFP) (reporter genes); and epidermal growth factor receptor (EGFR) genes lacking the intracellular domain. The detection gene is linked to a chimeric target factor receptor gene via, for example, a bicistronic control sequence (e.g., internal ribosome entry site (IRES)) or a sequence encoding a self-cleaving peptide. Examples of self-cleaving peptides include, but are not limited to, the 2A peptide (T2A) derived from *Thosea asigna* virus. For example, the following self-cleaving peptides are known: the 2A peptide derived from foot-and-mouth disease virus (FMDV) (F2A), the 2A peptide derived from equine rhinitis A virus (ERAV) (E2A), and the 2A peptide derived from Porcine teschovirus (PTV-1) (P2A).

The polynucleotide of the present invention may be a linear polynucleotide or a cyclic polynucleotide (e.g., a vector). The vector may be a plasmid vector or a viral vector. The vector may be, for example, a vector for cloning or a vector for expression. Examples of vectors for expression include vectors for prokaryotic cells, such as *Escherichia coli,* or actinomycetes, and vectors for eukaryotic cells, such as yeast cells, insect cells, or mammalian cells. More specifically, examples of viral vectors include retrovirus vectors, lentivirus vectors, adenovirus vectors, adeno-associated virus vectors, herpesvirus vectors, and Sendai virus vectors; and examples of non-viral vectors include various plasmid vectors, liposome vectors, positively charged liposome vectors (Felgner, P.L., Gadek, T.R., Holm, M., et al. Proc. Natl. Acad. Sci., 84: 7413-7417, 1987), YAC vectors, and BAC vectors.

The polynucleotide of the present invention includes not only DNA and RNA, but also known chemically modified DNA or RNA as described below. To prevent degradation by hydrolases, such as nucleases, the phosphate residue (phosphate) of each nucleotide may be replaced with, for example, a chemically modified phosphate residue, such as phosphorothioate (PS), methylphosphonate, or phosphorodithionate. The hydroxyl group at position 2 of a ribose of each ribonucleotide may be replaced with -OR (R represents, for example, CH₃ (2'-O-Me), CH₂CH₂OCH₃ (2'-O-MOE), CH₂CH₂NHC(NH)NH₂, CH₂CONHCH₃, or CH₂CH₂CN). Additionally, the base moiety (pyrimidine, purine) may be chemically modified by, for example, introduction of a methyl group or a cationic functional group into position 5 of the pyrimidine base, or replacement of the carbonyl group at position 2 with thiocarbonyl. Additionally, the polynucleotide of the present invention also includes, but is not limited to, those in which the phosphoric acid moiety or hydroxyl moiety is modified with biotin, an amino group, a lower alkyl amine group, an acetyl group, or the like. The term "polynucleotide" includes not only natural nucleic acids but also BNA (bridged nucleic acid), LNA (locked nucleic acid), and PNA (peptide nucleic acid).

In an embodiment of the present invention, an antigen-presenting cell that is activated in response to a target factor can be obtained by a method including introducing the polynucleotide of the present invention into an antigen-presenting cell. The cell expresses the chimeric target factor receptor of the present invention. Examples of the antigen-presenting cell into which the polynucleotide of the present invention is introduced include dendritic cells, macrophages, and precursor cells of these. Various cell populations can also be used as long as they include these cells.

### 4. Cell

In an embodiment, the present invention relates to a cell containing the polynucleotide of the present invention (which may be referred to as "the cell of the present invention" in the present specification). The following describes the cell.

Cells from which the cell of the present invention is derived are not particularly limited. If the cell of the present invention is intended for use in purification of the chimeric target factor receptor of the present invention, the origin of the cell can be, for example, those used in protein expression (e.g., insect cells, eukaryotic cells, and mammalian cells).

The cell of the present invention is preferably an antigen-presenting cell (e.g., a dendritic cell and a macrophage, particularly preferably a dendritic cell). In an embodiment, the cell of the present invention may be a stem cell that can be induced to differentiate into an antigen-presenting cell, such as an ES cell or an iPS cell. These cells are preferably cells expressing the chimeric target factor receptor of the present invention; in a more specific embodiment, these cells express the target factor receptor of the present invention on the cell membrane, and preferably express the target factor receptor of the present invention with the target factor-binding domain extracellularly exposed.

A cell such as an antigen-presenting cell expressing the chimeric target factor receptor recognizes a target factor at the target factor-binding domain, and then intracellularly transmits a recognition signal, thereby activating the cell itself. This regulates the activity of T cells (e.g., Th1 cells, Th2 cells, Th17 cells, and cytotoxic T cells), B cells, and like cells to develop anticancer activity, immunomodulatory activity etc. Therefore, a cell (in particular, an antigen-presenting cell) containing the polynucleotide encoding the chimeric target factor receptor of the present invention is useful as an active ingredient of a pharmaceutical composition.

### 5. Pharmaceutical Composition

In an embodiment, the present invention relates to a pharmaceutical composition containing the cell of the present invention (which may also be referred to as "the pharmaceutical composition of the present invention" in the present specification). The following describes the pharmaceutical composition.

The pharmaceutical composition of the present invention can be widely used as a cell formulation in the treatment, prevention, or amelioration of tumors or cancers expressing a target factor, in the treatment, prevention, or amelioration of autoimmune diseases, allergic diseases, etc., or in the treatment, prevention, or amelioration of infectious diseases. Cancers include solid cancers and hematological cancers. Examples of cancers include various B-cell malignant lymphomas (B-cell acute lymphocytic leukemia, follicular lymphoma, diffuse large-cell lymphoma, mantle cell lymphoma, MALT lymphoma, intravascular B-cell lymphoma, CD20 positive Hodgkin lymphoma), myeloproliferative diseases, myelodysplastic/myeloproliferative tumors (CMML, JMML, CML, MDS/MPN-UC), myelodysplastic syndrome, acute myeloid leukemia, multiple myeloma, lung cancer, colorectal cancer, ovarian cancer, breast cancer, brain tumor, stomach cancer, liver cancer, tongue cancer, thyroid cancer, kidney cancer, prostate cancer, uterine cancer, osteosarcoma, chondrosarcoma, and rhabdomyosarcoma. "Treatment" includes alleviating (moderating) symptoms characteristic to a target disease or concomitant symptoms, and inhibiting or delaying the worsening of symptoms. "Prevention" refers to preventing or delaying the onset or appearance of a disease (disorder) or symptoms of a disease, or reducing the risk of onset or appearance of a disease (disorder) or symptoms of a disease. "Amelioration" refers to alleviation (moderation), change for the better, remission, or cure (including partial cure) of a disease (disorder) or symptoms of a disease.

The pharmaceutical composition of the present invention contains a therapeutically effective amount of the cell of the present invention. For example, the pharmaceutical composition of the present invention for a single dose may contain the cell of the present invention in an amount of 1 × 10⁴ to 1 × 10¹⁰ cells. The cell formulation may contain dimethylsulfoxide (DMSO) or serum albumin for cell protection; antibiotics for preventing bacterial contamination; and various components for cell activation, proliferation, or inductive differentiation (e.g., vitamins, cytokines, growth factors, and steroids).

The administration route of the pharmaceutical composition of the present invention is not particularly limited. For example, the pharmaceutical composition of the present invention is administered by intravenous injection, intraarterial injection, portal venous injection, intradermal injection, subcutaneous injection, intramuscular injection, or intraperitoneal injection. Instead of systemic administration, local administration may be used. Examples of local administration include direct injection into a target tissue, a target organ, or a target body part. The administration schedule may be prepared in consideration of, for example, the sex, age, body weight, and pathological condition of the subject (patient). The pharmaceutical composition of the present invention may be administered in a single dose or in continuous or periodical multiple doses.

The pharmaceutical composition of the present invention or the cell of the present invention may be used in combination with other drugs. When used in combination with, in particular, an immune checkpoint inhibitor (e.g., anti-PD-1 antibodies, such as nivolumab and pembrolizumab, anti-PD-L1 antibodies, such as Tecentriq, durvalumab, and avelumab, and anti-CTLA-4 antibodies, such as ipilimumab), the pharmaceutical composition of the present invention or the cell of the present invention can improve their antitumor effect. In this respect, the pharmaceutical composition of the present invention and the cell of the present invention have application for use in combination with an immune checkpoint inhibitor, and the pharmaceutical composition of the present invention may contain an immune checkpoint inhibitor. The timing for combing these is not particularly limited. For example, the pharmaceutical composition of the present invention or the cell of the present invention may be administered at the same time with or on the same day of the administration of an immune checkpoint inhibitor; alternatively, one may be administered one or more days (e.g., 2 to 14 days) before or after the administration of the other.

### Examples

The present invention is described in detail below with reference to Examples. However, the present invention is not limited to these Examples.

### Test Example 1: Preparation of Chimeric Target Factor Receptor Construct

A chimeric target factor receptor construct containing the following disposed in this order from the N-terminus was designed:
- The signal peptide of GMCSFR (amino acid sequence: SEQ ID NO: 19, base sequence: SEQ ID NO: 20)
- Anti-Her2 scFv domain (amino acid sequence: SEQ ID NO: 1, base sequence: SEQ ID NO: 9)
- CD28 transmembrane domain (amino acid sequence: SEQ ID NO: 2, base sequence: SEQ ID NO: 10)
- Intracellular domain:
   (a) TLR1 intracellular domain (amino acid sequence: SEQ ID NO: 3, base sequence: SEQ ID NO: 11),
   (b) TLR2 intracellular domain (amino acid sequence: SEQ ID NO: 4, base sequence: SEQ ID NO: 12),
   (c) TLR3 intracellular domain (amino acid sequence: SEQ ID NO: 5, base sequence: SEQ ID NO: 13),
   (d) TLR4 intracellular domain (amino acid sequence: SEQ ID NO: 6, base sequence: SEQ ID NO: 14),
   (e) TLR9 intracellular domain (amino acid sequence: SEQ ID NO: 7, base sequence: SEQ ID NO: 15),
   (f) TLR2 Intracellular Domain - CD40 Intracellular Domain (amino acid sequence: SEQ ID NO: 8, base sequence: SEQ ID NO: 16),
   (g) TLR3 intracellular domain - CD40 intracellular domain,
   (h) TLR9 intracellular domain - CD40 intracellular domain,
   (i) CD40 intracellular domain - TLR2 intracellular domain,
   (j) CD40 intracellular domain - TLR3 intracellular domain, or
   (k) CD40 intracellular domain - TLR9 intracellular domain.

Additionally, a chimeric target factor receptor construct containing the following disposed in this order from the N-terminus was designed:
•The signal peptide of GMCSFR (amino acid sequence: SEQ ID NO: 19, base sequence: SEQ ID NO: 20)
•Anti-mesothelin (MSLN) scFv domain (amino acid sequence: SEQ ID NO: 17, base sequence: SEQ ID NO: 18)
•CD28 transmembrane domain
•Intracellular domain:
   (A) TLR1 intracellular domain,
   (B) TLR2 intracellular domain,
   (C) TLR3 intracellular domain,
   (D) TLR4 intracellular domain,
   (E) TLR9 intracellular domain,
   (F) TLR2 intracellular domain - CD40 intracellular domain,
   (G) TLR9 intracellular domain - CD40 intracellular domain,
   (H) CD40 intracellular domain,
   (I) CD40 intracellular domain - TLR9 intracellular domain, or
   (J) None.

The coding sequence of the chimeric target factor receptor and the coding sequence of the chimeric target factor receptor having the intracellular domain deleted were individually inserted into a pMXs-GFP vector (Cosmo Bio Co., Ltd.) to obtain respective pMXs-CAR-GFP vectors.

### Test Example 2: Preparation of Chimeric Target Factor Receptor-expressing Dendritic Cell

A pMXs-CAR-GFP vector (Test Example 1) was introduced into Plat-A retroviral packaging cells (Cell Biolabs, Inc.) using a TransIT-293 Reagent (Mirus LLC). After 48 hours, the culture supernatant was collected. The culture supernatant was added to THP-1 cells (obtained from the JCRB cell bank) to allow for viral infection. After 48 hours from the infection, GFP-positive cells were collected by using a BD FACSAria cell sorter. Human GM-CSF (BioLegend) and human IL-4 (BioLegend) were added to the obtained cells, and the cells were cultured for 5 days and differentiated into dendritic cells to obtain chimeric target factor receptor-expressing dendritic cells.

### Test Example 3: Preparation of Chimeric Target Factor Receptor-expressing Macrophage

A pMXs-CAR-GFP vector (Test Example 1) was introduced into Plat-A retroviral packaging cells (Cell Biolabs, Inc.) using a TransIT-293 Reagent (Mirus LLC). After 48 hours, the culture supernatant was collected. The culture supernatant was added to U937 cells (obtained from the JCRB cell bank) to allow for viral infection. After 48 hours from the infection, GFP-positive cells were collected by using a BD FACSAria cell sorter to obtain chimeric target factor receptor-expressing macrophages.

### Test Example 4: In Vitro Dendritic Cell Activation Assay 1

Chimeric target factor receptor-expressing dendritic cells (Test Example 2: the target factor is Her2) were added to Her2-expressing cells (SKOV3 cells: obtained from the ATCC) and cultured for 5, 24, and 48 hours, followed by investigating the expression of the index factors for dendritic cell activation (CD80 and CD86) by FACS.

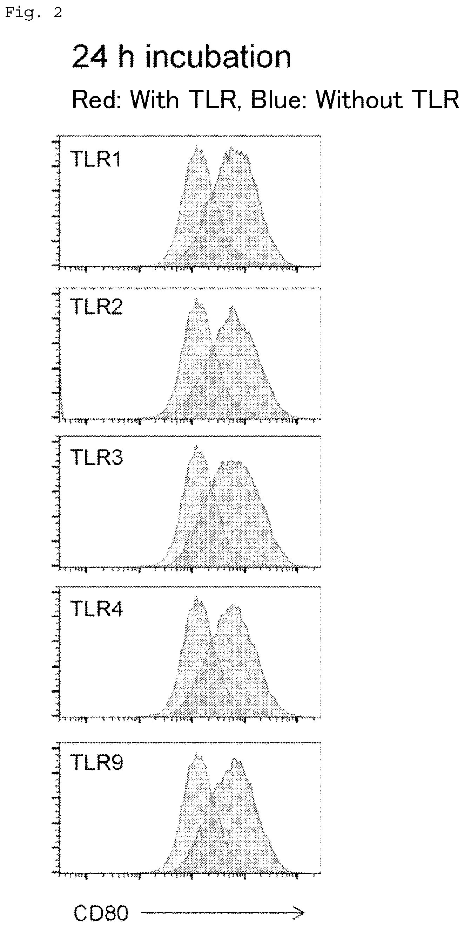

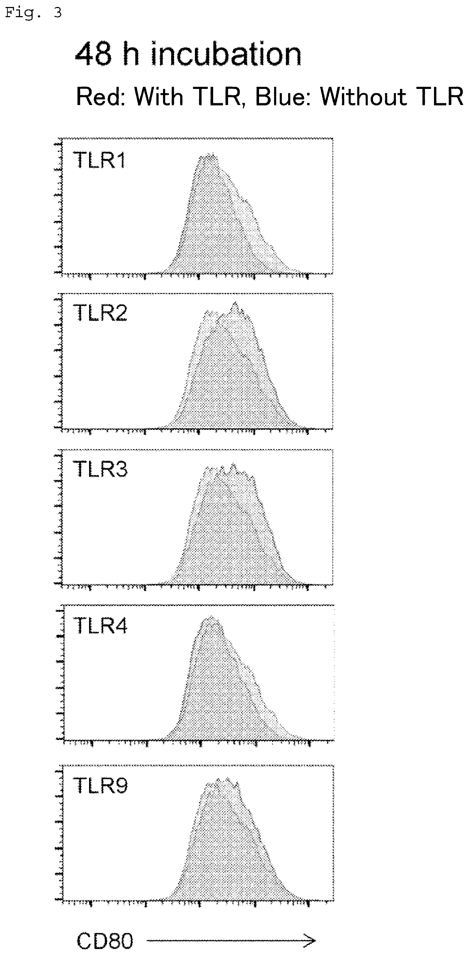

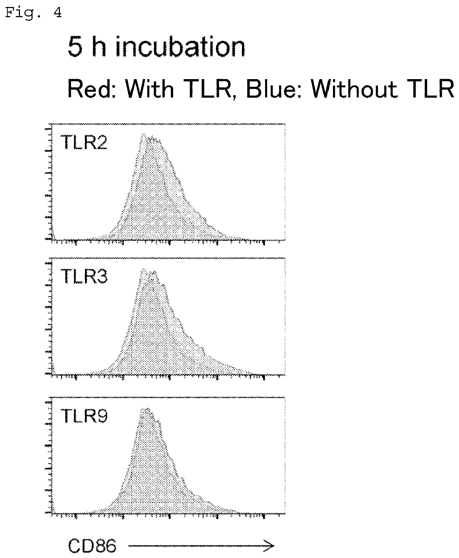

Figs. 1 to 5 show the results. As shown in Figs. 1 to 3, the results indicate that the use of the intracellular domain of TLR for the intracellular domain increases the expression of CD80 in response to the target factor. In particular, the results indicate that the use of the intracellular domains of TLR3 or TLR9 can increase the expression of CD80 at a relatively early stage (5 hours, Fig. 1), and that the use of the intracellular domain of TLR2, TLR3, or TLR9 can increase the expression of CD80 more persistently (48 hours, Fig. 3). Additionally, as shown in Fig. 4, the use of the intracellular domain of TLR for the intracellular domain was also confirmed to increase the expression of CD86 in response to the target factor. The use of the intracellular domain of CD40 in addition to the intracellular domain of TLR for the intracellular domain was also confirmed to increase the expression of CD86 more persistently (24 hours, Fig. 5) .

### Test Example 5: In Vitro Macrophage Phagocytosis Assay

Chimeric target factor receptor-expressing macrophages (Test Example 2: the target factor is Her2) were added to Her2-expressing cells (SKOV3 cells: obtained from the ATCC) and cultured for 48 hours, followed by investigating the phagocytic capacity by using a phagocytosis assay kit (Cayman).

Fig. 6 shows the results. As shown in Fig. 6, the results indicate that the use of the intracellular domain of TLR for the intracellular domain can also activate macrophages in response to the target factor.

### Test Example 6: Antitumor Effect Evaluation Test 1

### Test Example 6-1: Preparation of MSLN-expressing Mouse Tumor Cell Pan02/MSLN

The human MSLN gene was introduced into a pMXs-GFP vector. The obtained vector and a pCL-Eco vector (Addgene) were introduced into G3T-hi cells (Takara Bio, Inc.) by using a TransIT-293 Reagent (Mirus LLC). After 48 hours, the culture supernatant was collected. The culture supernatant was added to mouse pancreas cancer cells Pan02 to allow for viral infection. After 48 hours from the infection, GFP-positive cells were collected by using a BD FACSAria cell sorter.

### Test Example 6-2: Evaluation of Antitumor Effect

Pan02 or Pan02/MSLN (Test Example 6-1) was subcutaneously transplanted into mice, and dendritic cells (Empty: pMXs-GFP being introduced) or chimeric target factor receptor-expressing dendritic cells (Test Example 2: the target was MSLN, and the intracellular domain was (E) of Test Example 1) were administered, followed by examining their antitumor effects with tumor growth inhibition as an index. A tumor cut into 2-mm squares was transplanted subcutaneously into the flank of mice with a tumor-feeding needle. When the size of the tumor reached 100 mm³, the mice were divided into groups, and administration of cells was started. Cells (dendritic cells (Empty) or chimeric target factor receptor-expressing dendritic cells (MSLN-TLR9)) in an amount of 1 × 10⁵ cells were injected into the tumor once (Day 0). Tumor size was measured twice a week to evaluate the antitumor effect.

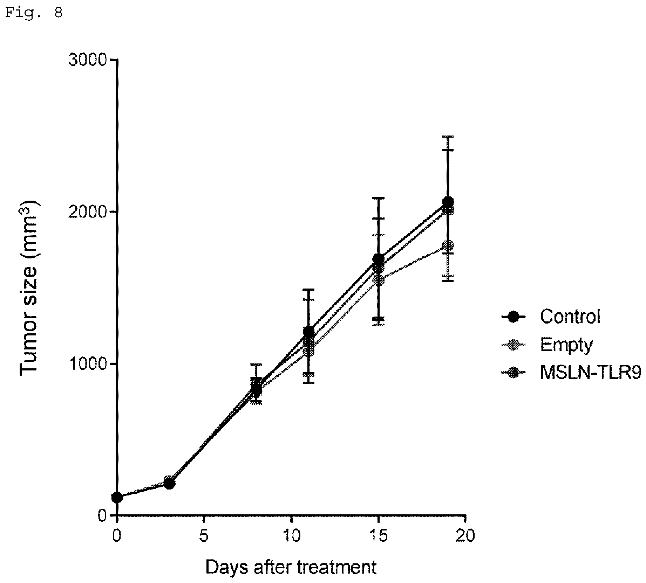

Figs. 7 and 8 show the results. As shown in Figs. 7 and 8, the results indicate that the use of the intracellular domain of TLR for the intracellular domain provides an antitumor effect in response to the target factor (MSLN).

### Test Example 7: In Vitro Dendritic Cell Activation Assay 2

Chimeric target factor receptor-expressing dendritic cells (Test Example 2: the target factor was Her2) were added to Her2-expressing cells (SKOV3 cells: obtained from the ATCC) and cultured for 24 hours, followed by investigating the expression of the index factors for dendritic cell activation (MHC class I and MHC class II) by FACS.

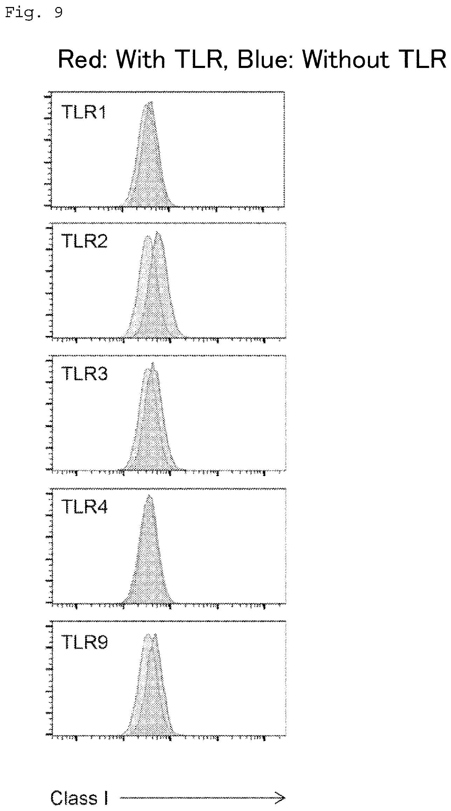

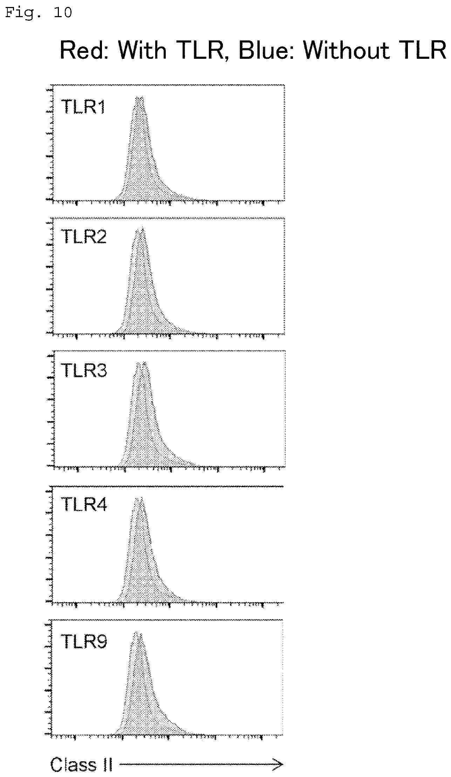

Figs. 9 and 10 show the results. The results indicate that the use of the intracellular domain of TLR for the intracellular domain can increase the expression of MHC class I and MHC class II in response to the target factor. In particular, the degree of activation was found to be high when TLR2, TLR3, or TLR9 was used (Fig. 9).

### Test Example 8. In Vitro Dendritic Cell Activation Assay 3

### Test Example 8-1: Preparation of MSLN-expressing Mouse Tumor Cell Panc-1/MSLN

The human MSLN gene was introduced into a pMXs-rCD2 vector. The obtained vector was introduced into Plat-A cells (Cell Biolabs, Inc.) by using a TransIT-293 Reagent (Mirus LLC). After 48 hours, the culture supernatant was collected. The culture supernatant was added to human pancreas cancer cells Panc-1 cells (obtained from the ATCC) to allow for viral infection. After 48 hours from the infection, rCD2-positive cells were collected by using a BD FACSAria cell sorter.

### Test Example 8-2: Activation Properties Evaluation Test

Chimeric target factor receptor-expressing dendritic cells (Test Example 2: the target factor was MSLN) were added to Panc-1/MSLN (Test Example 8-1) and cultured for 24 hours, followed by investigating the expression of the index factors for dendritic cell activation (CD86 and MHC class II) by FACS.

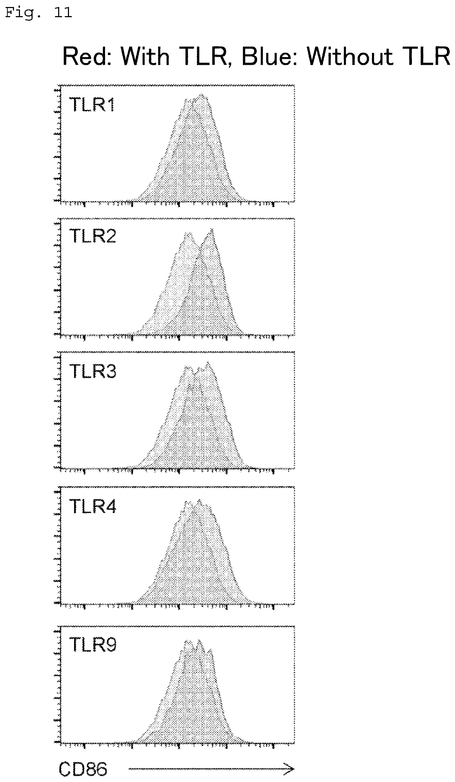

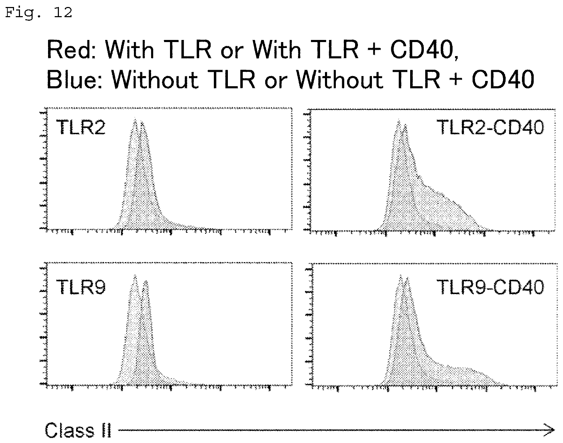

Figs. 11 and 12 show the results. The results indicate that the use of the intracellular domain of TLR for the intracellular domain can increase the expression of CD86 and MHC class II even when the target factor used was different from that of Test Example 4 or 8. Additionally, this test example also found that the use of the intracellular domain of CD40 in addition to the intracellular domain of TLR for the intracellular domain can increase the degree and persistence of dendritic cell activation.

### Test Example 9: Antitumor Effect Evaluation Test 2

Pan02 or Pan02/MSLN (Test Example 6-1) was subcutaneously transplanted into mice, and dendritic cells (Empty: pMXs-GFP being introduced) or chimeric target factor receptor-expressing dendritic cells were administered, followed by investigating their antitumor effect with tumor growth inhibition as an index. The target of the administered chimeric target factor receptor-expressing dendritic cells (Test Example 2) was MSLN, and the intracellular domain of the chimeric target factor receptor was any of the following: (A) to (E), (H), and (J) of Test Example 1. A tumor cut into 2-mm squares was transplanted subcutaneously into the flank of mice with a tumor-feeding needle. After 7 days from the transplantation, the mice were divided into groups, and administration of cells was started. Cells (dendritic cells (Empty) or chimeric target factor receptor-expressing dendritic cells) in an amount of 1 × 10⁵ cells were injected into the tumor once (Day 0). Tumor size was measured twice a week to evaluate the antitumor effect.

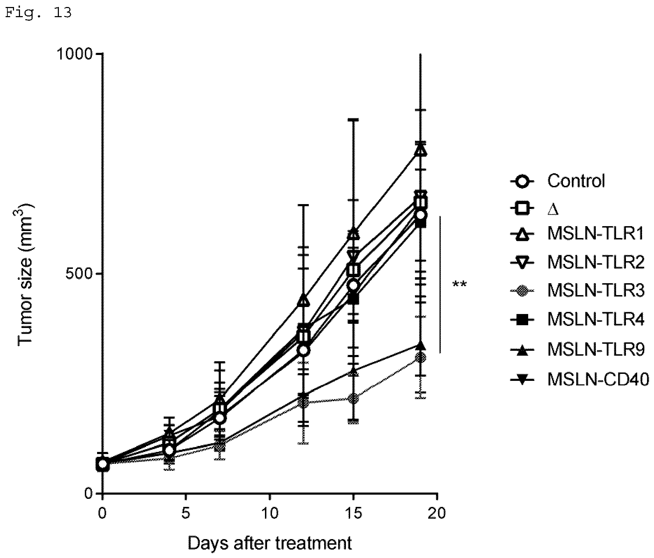

Fig. 13 shows the results. The results indicate that the use of the intracellular domain of TLR3 or TLR9 for the intracellular domain leads to a significantly higher antitumor effect in response to the target factor (MSLN) than the use of the intracellular domain of other TLRs.

### Test Example 10: Antitumor Effect Evaluation Test 3

Pan02 or Pan02/MSLN (Test Example 6-1) was subcutaneously transplanted into mice, and dendritic cells (Empty: pMXs-GFP being introduced) or chimeric target factor receptor-expressing dendritic cells were administered, followed by investigating their antitumor effect with tumor growth inhibition as an index. The target of the administered chimeric target factor receptor-expressing dendritic cells (Test Example 2) was MSLN, and the intracellular domain of the chimeric target factor receptor was (C) or (E) of Test Example 1. A tumor cut into 2-mm squares was transplanted subcutaneously into the flank of mice with a tumor-feeding needle. After 7 days from the transplantation, the mice were divided into groups, and administration of cells and/or a drug (PD-1 antibody) was started. For the group of mice for cell administration, 1 × 10⁵ cells (chimeric target factor receptor-expressing dendritic cells (MSLN-TLR9)) were administered into the tumor twice (Day 1 and Day 8). For the group for PD-1 antibody administration, 250 µg of a PD-1 antibody was administered intraperitoneally twice (Day 0 and Day 7). Tumor size was measured twice a week.

Tables 1 and 2 show the average measurement value of the tumor size of the group to which only cells were administered (TLR3 group or TLR9 group) and of the group to which only the PD-1 antibody (αPD-1 group) was administered, the average measurement value of the tumor size of the group to which cells were administered in combination with the PD-1 antibody (O-FTV), the tumor size in the case of combined administration assumed from the measured values of the TLR3 group or TLR9 group with the αPD-1 group (E-FTV (= the average measurement value of group 1 × the average measurement value of group 2), and the value obtained by dividing E-FTV by O-FTV (E/O). A value E/O of greater than 1 indicates that a synergistic effect was exhibited by the combined administration of the cells and the PD-1 antibody. Table 1 shows the results of the case in which the intracellular domain of the chimeric target factor receptor was the intracellular domain of TLR-3, and Table 2 shows the results of the case in which the intracellular domain of the chimeric target factor receptor was the intracellular domain of TLR-9. In the tables, the "Day" column indicates the number of days elapsed from Day 0 that is 7 days after transplantation.

**Table 1**

| | | | | TLR3 + αPD-1 | | |
|---|---|---|---|---|---|---|
| Day | TLR3 | αPD-1 | TLR3 + αPD-1 | E-FTV | O-FTV | E / O |
| 13 | 0.568 | 0.774 | 0.409 | 0.439 | 0.409 | 1.076 |
| 16 | 0.494 | 0.770 | 0.342 | 0.381 | 0.342 | 1.112 |
| 20 | 0.493 | 0.758 | 0.357 | 0.373 | 0.357 | 1.046 |

**Table 2**

| | | | | TLR9 + αPD-1 | | |
|---|---|---|---|---|---|---|
| Day | TLR9 | αPD-1 | TLR9 + αPD-1 | E-FTV | O-FTV | E / O |
| 20 | 0.603 | 0.758 | 0.433 | 0.457 | 0.433 | 1.056 |

As shown in Tables 1 and 2, the results indicate that the use of chimeric target factor receptor-expressing cells containing a TLR intracellular domain in combination with a PD-1 antibody synergistically improves the antitumor effect.

### Test Example 11: Antitumor Effect Evaluation Test 4

Pan02 or Pan02/MSLN (Test Example 6-1) was subcutaneously transplanted into mice, and dendritic cells (Empty: pMXs-GFP being introduced) or chimeric target factor receptor-expressing dendritic cells were administered, followed by investigating their antitumor effect with tumor growth inhibition as an index. The target of the administered chimeric target factor receptor-expressing dendritic cells (Test Example 2) was MSLN, and the intracellular domain of the chimeric target factor receptor was (E), (F), or (I) of Test Example 1. A tumor cut into 2-mm squares was transplanted subcutaneously into the flank of mice with a tumor-feeding needle. After 7 days from the transplantation, the mice were divided into groups, and administration of cells was started. Cells (dendritic cells (Empty) or chimeric target factor receptor-expressing dendritic cells) in an amount of 1 × 10⁵ cells were injected into the tumor once (Day 0). Tumor size was measured twice a week to evaluate the antitumor effect.

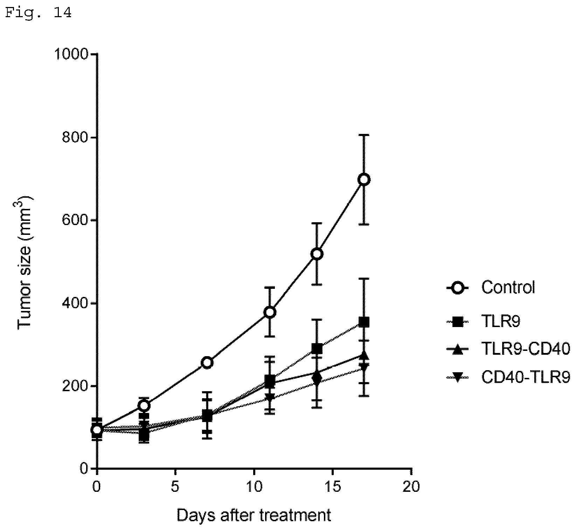

Fig. 14 shows the results. The results indicate that the use of the intracellular domain of CD40 in addition to the intracellular domain of TLR for the intracellular domain improves the antitumor effect.

### Sequence Listing

P21-207WO_ PCT_Chimeric Target Factor
Receptor_20211130_131343_2.txt

## Claims

1. A chimeric target factor receptor comprising
a target factor-binding domain,
a transmembrane domain, and
an intracellular domain containing a TLR intracellular domain.

2. The chimeric target factor receptor according to claim 1, wherein the TLR intracellular domain is at least one selected from the group consisting of a TLR2 intracellular domain, a TLR3 intracellular domain, and a TLR9 intracellular domain.

3. The chimeric target factor receptor according to claim 1 or 2, wherein the TLR intracellular domain is at least one selected from the group consisting of a TLR3 intracellular domain and a TLR9 intracellular domain.

4. The chimeric target factor receptor according to any one of claims 1 to 3, wherein the intracellular domain further contains a CD40 intracellular domain.

5. The chimeric target factor receptor according to any one of claims 1 to 4, wherein the target factor-binding domain has a single-chain antibody structure.

6. The chimeric target factor receptor according to any one of claims 1 to 5, wherein the target factor is at least one selected from the group consisting of cancer antigens, cytokine receptors, and cell growth factor receptors.

7. A polynucleotide encoding the chimeric target factor receptor of any one of claims 1 to 6.

8. A cell comprising the polynucleotide of claim 6.

9. The cell according to claim 8, which is an antigen-presenting cell.

10. The cell according to claim 8 or 9, which is a dendritic cell.

11. A pharmaceutical composition comprising the cell of any one of claims 8 to 10.

12. The pharmaceutical composition according to claim 11, which is for use in the treatment, prevention, or amelioration of at least one selected from the group consisting of cancers, autoimmune diseases, allergic diseases, and infectious diseases.

13. The pharmaceutical composition according to claim 11 or 12, which is for use in the treatment, prevention, or amelioration of solid cancer.

14. The pharmaceutical composition according to claim 13, wherein
the TLR intracellular domain is at least one selected from the group consisting of a TLR3 intracellular domain and a TLR9 intracellular domain, and
the cell is a dendritic cell.

15. The pharmaceutical composition according to claim 13 or 14, wherein
the intracellular domain further contains a CD40 intracellular domain, and
the cell is a dendritic cell.

16. The pharmaceutical composition according to any one of claims 11 to 15, which is for use in combination with an immune checkpoint inhibitor.
